# EUROPEAN PATENT APPLICATION

(11) **EP 1 287 809 A1**
(43) Date of publication of application: **05.03.2003**
(21) Application number: 02255894.4
(22) Date of filing: 23.08.2002
(51) Int. Cl.: A61K 7/032, A61K 7/48

(54) **Reduced-wax mascara compositions**

(30) Priority: 24.08.2001 US 938454
(71) Applicant: NEUTROGENA CORPORATION, Los Angeles California 90045-5544 (US)
(72) Inventor: Collins, Carol J., Granda Hills, CA 91344 (US); Woodin, Frederick W., Redondo Beach, CA 90277 (US); Villamor, Minerva, Gardena, CA 90247 (US)
(74) Representative: Fisher, Adrian John

(57) **Abstract**

The present invention relates to a mascara that includes a silicone gel that contains a polysiloxane elastomer and a silicone oil, wherein the mascara includes less than about 1%, by weight, of wax.

## Description

### FIELD OF THE INVENTION

The present invention relates to a mascara composition.

### BACKGROUND OF THE INVENTION

Mascaras are used to color, thicken, and accentuate the eyelashes. To obtain these goals, most mascara compositions contain waxes as a means to obtain all of these attributes. However, waxes provide certain undesirable properties. The resulting mascaras often feel heavy, sticky and stiff on the lashes, make lashes dry and brittle, easily smudge and flake, and make the eyelashes clump and stick together. As discussed in PCT Patent Application WO 00/74519, mascaras containing waxes also increase in viscosity during storage. Mascara manufactures, therefore, are faced with products that have a reduced shelf life.

Thus, it is desired to formulate mascaras that contain reduced levels of wax, or no wax at all, that still deliver the benefits of increasing volume and length to the lashes, and provide prolonged color adhesions while also minimizing or eliminating the above problems associated with wax mascaras.

Applicants have surprisingly found a mascara that contains a silicone gel that despite the reduction or absence of wax is able to color and thicken the eyelashes.

### SUMMARY OF THE INVENTION

The invention features a mascara that includes a silicone gel that contains a polysiloxane elastomer and a silicone oil, wherein the mascara includes less than about 1%, by weight, of wax.

Other features and advantages of the present invention will be apparent from the detailed description of the invention and from the claims

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference.

### Mascara

What is meant by the term "mascara" is a product that contains a pigment for use in depositing such a pigment onto eyelashes and/or eyebrows. In one embodiment, the mascara visibly increases the volume of the eyelash or eyebrow by adhering to the eyelashes and/or eyebrow.

In one embodiment, the mascara is an emulsion containing an aqueous phase, a non-aqueous phase, and one or more emulsifiers. What is meant by the term "non-aqueous phase" is a phase containing one or more ingredients that are not soluble in the aqueous phase of the composition, including, but not limited to lipophilic materials such as oils. In a further embodiment, the non-aqueous phase contains the silicone gel.

The mascara of the present invention comprises less than about 1%, by weight, of wax. What is meant by the term "wax" is an animal (e.g., fatty acid esters), mineral (e.g., parafinic oils), vegetable (e.g., vegetable oils), or synthethic hydrocarbons that are solid at room temperature, soluble in organic solvents, and substantially not soluble in water (e.g., less than 0.1 mg/ml at 25°). Waxes include animal waxes, plant waxes, mineral waxes, and petroleum waxes. Examples of waxes include, but are not limited to, beeswax, paraffin wax, ozokerite, candelilla wax, cetyl alcohol, stearyl alcohol, spermaceti, carnauba wax, baysberry wax, montan, ceresin, and microcrystalline waxes. Other examples of waxes are found on pages 1604-05 of the International Cosmetic Ingredient Dictionary and Handbook (7^{th} Ed., The Cosmetic, Toiletry, and Fragrance Association, Washington, DC, 1997), hereinafter the ICI Handbook. In one embodiment, the mascara comprises a total amount of less than about 0.5%, by weight, of wax. Preferably, the mascara does not comprise any wax.

### Silicone Gel

The silicone gel of the present invention includes one or more organopolysiloxane elastomer(s) and one or more silicone oil(s). In one embodiment, the organopolysiloxane elastomer is dispersed in the silicone oil, for example, using standard homogenization techniques.

Organopolysiloxane elastomer compounds are chain polymers having a degree of cross-linking sufficient to provide a rubber-like material. Suitable organopolysiloxane elastomers are disclosed in U.S. Pat. No. 5, 266,321 and 5,412,004, and the disclosure of which is incorporated herein by reference. The organopolysiloxane of the present invention have a three dimensional cross-linked structure and may have an average molecular weight in excess of 10,000 (e.g., between about 10,000 and 10,000,000). Examples of silicone polymers include crosslinked siloxane copolymers such as stearyl methyl-dimethyl siloxane copolymer (Gransil SR-CYC, available from Grant Industries, Elmwood Park, NJ); Polysilicone-11 (i.e., a crosslinked silicone rubber formed by the reaction of vinyl terminated silicone and methylhydrodimethyl siloxane in the presence of cyclomethicone also available from Grant Industries), cetearyl dimethicone/vinyl dimethicone crosspolymer (i.e., a copolymer of cetearyl dimethicone crosslinked with vinyl dimethyl polysiloxane), dimethicone/phenyl vinyl dimethicone crosspolymer (i.e., copolymer of dimethylpolysiloxane crosslinked with phenyl vinyl dimethylsiloxane), and dimethicone/vinyl dimethicone crosspolymer (i.e., copolymer of dimethylpolysiloxane crosslinked with vinyl dimethylsiloxane).

In one embodiment, the silicone oil(s) is a volatile silicone oil including, but not limited to, a low molecular weight organopolysiloxane. Representative volatile organopolysiloxanes include, but are not limited to, cyclomethicone and lower molecular weight dimethicones or mixtures thereof. Particularly preferred as volatile silicone oils are cyclomethicones such as cyclic organpolysiloxanes having ring sizes of 4 to 12, such as cyclotetrasiloxane and cyclopentasiloxane, and methylated cyclic organpolysiloxanes having ring sizes of 4 to 12, such as octamethylcyclotetrasiloxane and decamethycyclopentasiloxane, and mixtures thereof. Such silicones generally have a molecular weight of about 100 to 400 and are available commercially from Dow Corning Corporation, Mount Olive, N.J. and GE Silicones, Waterford, N.Y.

In one embodiment, the silicone oil(s) is a non-volatile silicone oil, including, but not limited to, a high molecular weight organopolysiloxane. Representative volatile organopolysiloxanes include, but are not limited to, methylated linear polysiloxanes such as higher molecular weight dimethicones; alkylated derivatives of linear polysiloxanes such as cetyl dimethicone and lauryl trimethicone; hydroxylated derivatives of linear polysiloxanes such as dimethiconol; and mixtures thereof. Such silicones generally have a molecular weight of about 100,000 to 1,000,000 and are available commercially from Dow Corning Corporation and GE Silicones.

The silicone gel can be comprised of a blend or mixture of two or more silicone oils, and may be a blend or mixture of a volatile silicone oil and a non-volatile silicone oil.

Such silicone gels are also commercially available from Grant Industries under the trade names Gransil GCM-5, Gransil DMCM-5, and Gransil DMG-20P

### Viscosity Increasing Agents

In addition to the silicone gel, the present composition may further comprise a viscosity increasing agent such as micas, hydroxyethylcellulose, carbomer, and bentonite. Examples of micas, include, but are not limited to, aluminum silicates such as aluminum fluoro megnesium sodium silicate and aluminum flouro magnesium potassium silicate. Other examples of viscosity increasing agents can be found on pages 1693-97 of the ICI Handbook.

### Film Forming Agent

In addition to the silicone gel, the present composition may further comprise a film forming agent. Examples of film forming agents include, but are not limited to, polyurathanes such as Polyurethane-2, Polyurethane-4, and Polyurethane-5, polyisobutene, polyvinyl alcohol, dimethicone copolyol polyacrylate, polyvinyl pyrrolidone (PVP), PVP/hexaecane copolymer, and PVM/MA copolymer. Other examples of film forming agents can be found on pages 1636-38 of the ICI Handbook.

### Pigments

The mascara of the invention comprises from about 0.1% to about 40%, such as about 1% to about 30%, by weight, of one or more pigments. The pigments may be colored or non-colored (for example white). The pigments may also be either organic or inorganic pigments. Examples of organic pigments include, but are not limited to, various aromatic pigments including azo, indigoid, triphenylmethane, anthraquinone, and xanthine dyes which are designated as D&C and FD&C colors (e.g., blues, greens, oranges, reds, violet, and yellows). Organic pigments generally consist of insoluble metallic salts of certified color additives, referred to as the Lakes, in particular the Lakes of D&C and FD&C colors. Inorganic pigments include iron oxides, ultramarines, chromium, chromium hydroxide colors, and mixtures thereof. Other pigments are listed on pages 1628-30 of the ICI Handbook.

### Other Ingredients

The mascara may further comprise one or more humectants such as propylene glycol and glycerin, emulsifiers such as polysorbate 20, anti-foaming agents such a simithicone, pH adjusters such as citric acid, vitamins, and preservatives such as parabens and phenoxyethanol. Examples of such are disclosed on pages 1611, 1650-51, 1653-55, and 1679-86 of the ICI Handbook.

### Container

In one embodiment, the mascara of the present invention is contained within a container. In a further embodiment, the container further comprises a brush applicator for applying the mascara to eyelashes or eyebrows. In one embodiment, the brush applicator is affixed to the cap of the container. In one embodiment, the container is designed to displace excess mascara from the brush into the container upon removal of the brush from the container. Examples of mascara containers and brush applicators are well known in the art. Examples of such are set forth in PCT Patent Applications WO01/141,599, WO01/128,383, WO01/15510, WO94/1775, WO 93/11683, WO92/11785, and WO87/06807.

Other features and advantages of the present invention will be apparent from the detailed description of the invention and

The following is a description of the manufacture of mascaras of the present invention. Other compositions of the invention can be prepared in an analogous manner by a person of ordinary skill in the art.

### Example 1:

Mascaras of Table 1 can be manufactured in the following manner:

**Table 1**

| Item # | CHEMICAL NAME (INCI) | Function | % (w/w) |
|---|---|---|---|
| 1 | Water | Humectant/ Diluent | q.s. |
| 2 | Glycerin | Humectant | 0.01 - 20 |
| 3 | Propylene Glycol | Humectant | 0.01 - 20 |
| 4 | Polysorbate 20 | Surfactant/ Emulsifier | 0.01 - 10 |
| 5 | Simethicone | Anti-foam Agent | 0.01 - 10 |
| 6 | Mica | Viscosity Increasing Agent | 0.01 - 30 |
| 7 | Iron Oxide | Colorant/ Pigment | 0.01 - 20 |
| 8 | Dimethicone Copolyol Polyacrylate | Film Former | 0.01 - 10 |
| 9 | Hydroxyethylcellulose | Viscosity Increasing Agent | 0.01 - 10 |
| 10 | Polyurethane-1 | Film Forming Agent | 0.01 - 15 |
| 11 | Citric acid | pH Adjuster | 0.01 - 1 |
| 12 | Plant Extract | Aesthetic Ingredient | 0.01 - 5 |
| 13 | Cyclopentasiloxane and Polysilicone-11 | Conditioning Agent | 0.01 - 50 |
| 14 | Polyisobutene | Film Forming Agent | 0.01 - 20 |
| 15 | PVP/Hexadecene Copolymer | Film Forming Agent | 0.01 - 10 |
| 16 | Vitamins | Vitamin | 0.01 - 5 |
| 17 | Mica | Viscosity Increasing Agent | 0.01 - 30 |
| 18 | Hydrolyzed Wheat Protein | Conditioning Agent | 0.01 - 5 |
| 19 | Preservatives | Preservative | 0.01 - 5 |

Into a main kettle, part of the water and items 3, 4, 5, and 6 were added and mixed at moderate speed. Item 7 was then added and homogenized for until color was fully dispersed. Item 8 was then added and mixed until uniform.

In a first beaker, the remainder of the water and items 2 and 9 were added and mixed until the solids were dissolved and the resulting mixture was then added to the main kettle and mixed for about 20 minutes. The temperature was then increased to 50-55°C and mixing was continued until item 9 was fully hydrated. The mixture was then cooled down to 42-45°C following which items 10,11 and 12 were then added.

In a second beaker, item 13-16 were mixed and heated to 42-45°C until homogenous, and then added to the main kettle. The resulting mixture was mixed until uniform. Item 17 was then added until it swells and becomes smooth, following which items 18 and 19 were added. The mixture is then cooled to 32-35°C. The mixture is then filtered through a mesh filter

### Example 2:

The following mascara of Table 2 was manufactures as follows:

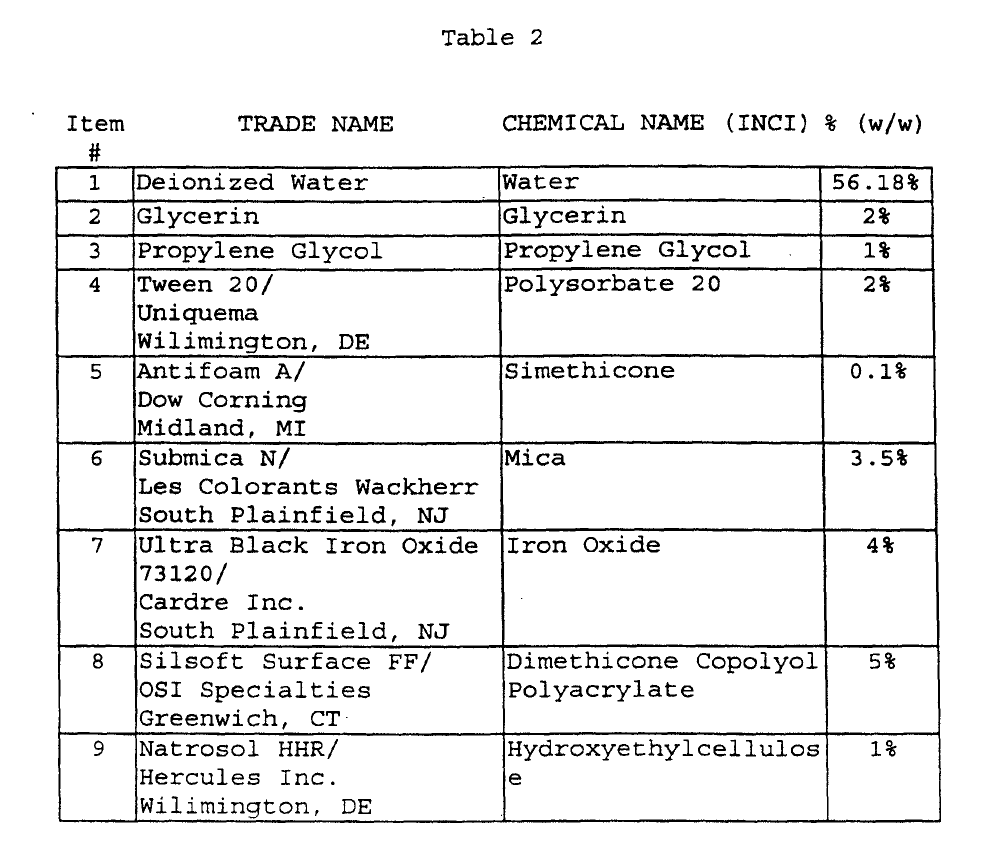

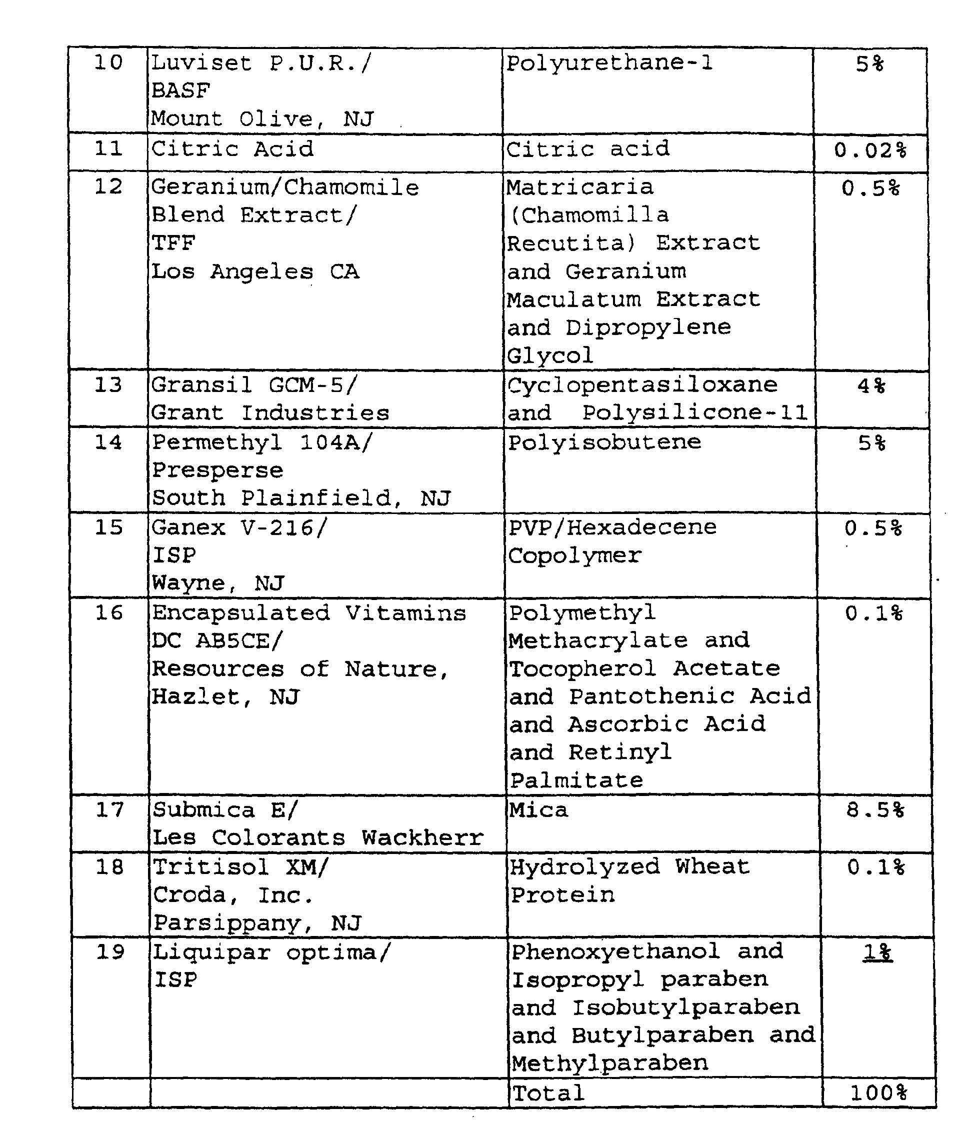

## Claims

1. A mascara comprising a silicone gel that comprises an organopolysiloxane elastomer and a silicone oil, wherein said mascara comprises less than about 1%, by weight, of wax.

2. A mascara of claim 1 wherein said mascara further comprises mica.

3. A mascara of claim 1 or claim 2 wherein said mascara further comprises a polyurethane.

4. A mascara of any preceding claim wherein said silicone oil is a cyclomethicone.

5. A mascara of claim 1 wherein said polysiloxane elastomer is polysilicone-11.

6. A mascara of claim 2 wherein said polysiloxane elastomer is polysilicone-11.

7. A mascara of claim 3 wherein said polysiloxane elastomer is polysilicone-11.

8. A mascara of claim 4 wherein said polysiloxane elastomer is polysilicone-11.

9. A mascara of any preceding claim wherein said mascara does not comprise wax.
